# EUROPEAN PATENT SPECIFICATION

(11) **EP 2 753 292 B1**
(45) Date of publication and mention of the grant of the patent: **27.06.2018**
(21) Application number: 12750748.1
(22) Date of filing: 17.08.2012
(51) Int. Cl.: A61K 8/02, A61K 8/24, A61K 8/25, A61K 8/365, A61K 8/44, A61Q 11/00

(54) **TOOTH REMINERALIZING DENTIFRICE**
ZAHNREMINERALISIERENDE ZAHNPASTA
DENTIFRICE DE REMINÉRALISATION DENTAIRE

(30) Priority: 08.09.2011 WO PCT/CN2011/001519
(43) Date of publication of application: 16.07.2014
(73) Proprietor: Unilever N.V., 3013 AL Rotterdam (NL); Unilever PLC, London, Greater London EC4Y 0DY (GB)
(72) Inventor: DENG, Yan, Shanghai 200335 (CN); HU, Qinghong, Shanghai 200335 (CN); LI, Li, Hangzhou Zhejiang 310058 (CN); TANG, Ruikang, Hangzhou Zhejiang 310058 (CN)
(74) Representative: Keenan, Robert Daniel
(86) International application number: PCT/EP2012/066120
(87) International publication number: WO 2013/034421

(56) References cited:
- EP-A1- 0 029 332
- EP-A1- 1 072 253
- WO-A1-00/78270
- WO-A1-2006/050966
- WO-A1-2007/137606
- DE-A1-102008 014 225
- JINHUI TAO ET AL: "Roles of Amorphous Calcium Phosphate and Biological Additives in the Assembly of Hydroxyapatite Nanoparticles", THE JOURNAL OF PHYSICAL CHEMISTRY B, vol. 111, no. 47, 3 November 2007 (2007-11-03), pages 13410-13418, XP055089585, ISSN: 1520-6106, DOI: 10.1021/jp0732918 cited in the application
- LI LI ET AL: "Bio-Inspired Enamel Repair via Glu-Directed Assembly of Apatite Nanoparticles: an Approach to Biomaterials with Optimal Characteristics", ADVANCED MATERIALS, vol. 23, no. 40, 25 October 2011 (2011-10-25), pages 4695-4701, XP055090159, ISSN: 0935-9648, DOI: 10.1002/adma.201102773
- LI LI ET AL: "Repair of enamel by using hydroxyapatite nanoparticles as the building blocks", JOURNAL OF MATERIALS CHEMISTRY, vol. 18, no. 34, 18 July 2008 (2008-07-18) , page 4079, XP055090249, ISSN: 0959-9428, DOI: 10.1039/b806090h

## Description

### TECHNICAL FIELD OF THE INVENTION

The present invention relates to dentifrices such as tooth pastes and mouthwashes. In particular the present invention relates to dentifrice compositions having a specific pH and comprising water insoluble and/or slightly soluble calcium source and organic acid. The invention also relates to the use of such dentifrices for remineralization and/or whitening of teeth.

### BACKGROUND TO THE INVENTION

Products we enjoy as consumers, unfortunately, often have a negative impact on our teeth. Acidic drinks and sweets, for example, can result in tooth erosion by attacking enamel that coats and protects the teeth. Moreover, tobacco based products as well as beverages like coffee and tea can stain teeth and thereby result in a smile that is often not attractive.

In addition to what we consume, the natural equilibrium between tooth hydroxyapatite (HAP) being dissolved from the enamel of teeth and HAP being formed on or in teeth from substances occurring naturally in the saliva shifts continuously. Such a shift can yield unattractive teeth with cariogenic conditions. Products that address tooth decay and/or whitening have, nevertheless, been developed. Conventional products often comprise peroxides, coarse abrasives or both.

International patent application published as WO 2007/066837 (LG HOUSEHOLD AND HEALTH CARE LTD) discloses a liquid delivery system for a teeth whitening ingredient. The whitening agent can be selected from peroxides, perborates, percarbonates, peroxyacids, persulfates and metal chlorites.

These types of products are often not desired since they can cause damage to teeth and gums if improperly used. Moreover, such products can be expensive and are not attractive to lower income consumers in need of tooth remineralization.

Cement-like restorative compositions have also been developed for use as tooth fillings or which otherwise solidify in or around teeth. Such compositions remain in contact with teeth for extended periods and are said to aid mineralization and/or whitening.

International patent application published as WO 2010/042754 (MED COLLEGE GEORGIA RES INST) discloses systems, methods, compositions and kits for mineralizing tissue, particularly dental tissue. The methods, compositions and kits may be used to strengthen and prevent weakening of the tissue. For example, the methods, compositions and kits may be used for treating and filling cavities in teeth caused by tooth decay. Japanese patent application published as JP 11-116421 A (LION CORP) discloses compositions capable of removing stains easily and operably, whitening teeth, and also preventing teeth from getting breakable by including a self-curing calcium phosphate compound, a fluorine compound, a peroxide, and a specific acid. EP0029332 is concerned with remineralisation of teeth and discloses dentifrices comprising hydroxyapatite, CMC sodium and carrageenin. Glutamine, proline, serine or glycine or mixtures thereof can be additionally present. Unfortunately such compositions may still require the use of harsh oxidizing agents, such as peroxides and/or require the entire solid composition to remain in contact with the teeth for extended periods of time. Thus such compositions may not be suitable to use in a consumer's daily routine of cleaning teeth for a few minutes with dentifrice.

The present inventors have therefore recognized that there is a need to develop an oral care product that is suitable to whiten and remineralize teeth while at the same time being gentle for use and/or affordable for a broad range of consumers and/or effective when used as part of daily teeth-cleaning or mouth-washing routines. This invention, therefore, is directed to a dentifrice composition as well as a method for remineralizing and/or whitening teeth.

### TESTS AND DEFINITIONS

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purposes of the present invention means a paste or gel dentifrice for use with a toothbrush. Especially preferred are tooth pastes suitable for cleaning teeth by brushing for about two minutes.

### Mouth Wash

"Mouth wash" for the purposes of the present invention means liquid dentifrice for use in rinsing the mouth. Especially preferred are mouth washes suitable for rinsing the mouth by swishing and/or gargling for about half a minute before expectorating.

### Solubility

"Soluble" and "insoluble", as used herein, refers to the solubility of a source (e.g., like calcium salts) in water at 25 °C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of greater than 0.001 moles per litre and less than 0.1 moles per litre.

### Particle Size

"Particle size" as used herein means diameter size and reported as a weight average particle size. "Diameter" is meant to mean the longest length measurable in any dimension in the event the particle is not a perfect sphere. Particle size can be measured, for example by dynamic light scattering (DLS).

### Remineralization and Whitening

"Remineralization", as used herein, means *in situ* (i.e. in the oral cavity) generation of hydroxyapatite (HAP) on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new HAP. New HAP, including layers thereof, typically covers at least 30 percent, and preferably, at least 45 percent and most preferably from 48 to 100 percent of the surface of teeth cleaned with the dentifrice composition of this invention and including all ranges subsumed therein. "Whitening" can also include the physical whitening of teeth through the adhesion of calcium-based salts and other opacifiers temporarily to the surface of teeth.

### pH

When referring to the pH of a composition, this means the pH measured when 1 part by weight of the composition is uniformly dispersed and/or dissolved in 20 parts by weight pure water at 25 °C. In particular the pH may be measured by manually mixing 1 g dentifrice with 20 ml water for 30s, then immediately testing the pH with indicator paper or a pH meter.

### Oxidative Whitening Compound

"Oxidative whitening compound", as used herein, means one or more of peroxides, perborates, percarbonates, peroxyacids, persulfates and metal chlorites.

### Substantially Free

"Substantially free of', as used herein, means less than 1.5%, and preferably less than 1.0%, and more preferably less than 0.75% and more preferably still less than 0.5% and most preferably from 0.0 to 0.1 % by weight, based on total weight of the dentifrice composition, including all ranges subsumed therein.

### Viscosity

Viscosity of a tooth paste is the value taken at room temperature (25 °C) with a Brookfield Viscometer, Spindle No. 4 and at a speed of 5 rpm. Values are quoted in centipoises (cP = mPa.s) unless otherwise specified.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final dentifrice composition, unless otherwise specified.

It should be noted that in specifying any range of values, any particular upper value can be associated with any particular lower value.

For the avoidance of doubt, the word "comprising" is intended to mean "including" but not necessarily "consisting of' or "composed of'. In other words, the listed steps or options need not be exhaustive.

### SUMMARY OF THE INVENTION

The present invention provides a dentifrice composition comprising:
(a) water insoluble and/or slightly soluble calcium source, wherein the calcium source comprises
   i. a component capable of reacting with phosphate ions to produce a calcium phosphate *in situ,*
   ii. hydroxyapatite (HAP) and/or amorphous calcium phosphate (ACP) having a weight average particle size of 5 microns or less, or
   iii. both; and
(b) at least 0.1% by weight of organic acid having 1 to 3 carboxylic acid groups,
   or its physiologically acceptable salt, or a combination wherein the organic acid is acidic amino acid; and
(c) surfactant, thickener humectant or a combination thereof; wherein the dentifrice composition has a pH of greater than 6.0; and wherein the component capable of reacting with phosphate ions to produce a calcium phosphate in situ comprises calcium oxide, calcium silicate, calcium carbonate, calcium hydroxide, calcium sulfate, calcium carboxymethylcellulose, calcium alginate, bioactive glass or a mixture thereof.
The composition is found to be surprisingly effective at remineralizing dental tissue, such as enamel and/or dentin when in normal use as a dentifrice and without any special procedures or implements. Thus further aspects of the invention relate to methods and uses which employ the composition for remineralizing and/or whitening of teeth of an individual.

### BRIEF DESCRIPTION OF THE FIGURES

Certain embodiments of the invention are illustrated by the figures, in which:
Fig. 1 is an SEM image of acid etched native enamel surface before incubation;
Fig. 2 is an SEM image of enamel surface after incubation in simulated body fluid (SBF) containing calcium silicate particles;
Fig. 3 is an SEM image of enamel surface after incubation in SBF containing calcium silicate particles and 100 mM glutamic acid;
Fig. 4 is an SEM image of a cross-section of an enamel block after brushing with anhydrous toothpaste containing calcium silicate particles for two weeks;
Fig. 5 is an SEM image of a cross-section of an enamel block after brushing for two weeks with the same anhydrous toothpaste as the sample in Fig. 4 but additionally containing 1% glutamic acid;
Fig. 6 is an SEM image of a cross-section of an enamel block after brushing for two weeks with the same anhydrous toothpaste as the sample in Fig. 4 but additionally containing 2% glutamic acid;
Fig. 7 is an SEM image of a cross-section of an enamel block after brushing for two weeks with the same anhydrous toothpaste as the sample in Fig. 4 but additionally containing 1% glycine;
Fig. 8 is an SEM image of a cross-section of an enamel block after brushing for two weeks with the same anhydrous toothpaste as the sample in Fig. 4 but additionally containing 1% citric acid;
Fig. 9 is an SEM image of a cross-section of an enamel block after brushing with hydrous toothpaste containing calcium silicate particles for four weeks;
Fig. 10 is an SEM image of a cross-section of an enamel block after brushing for four weeks with the same hydrous toothpaste as the sample in Fig. 9 but additionally containing 1% glycine; and
Fig. 11 is an SEM image of a cross-section of an enamel block after brushing for four weeks with the same hydrous toothpaste as the sample in Fig. 9 but additionally containing 2% glutamic acid.

### DETAILED DESCRIPTION

The composition of the present invention comprises a water insoluble and/or slightly soluble calcium source. The use of a water insoluble and/or slightly soluble calcium source allows for a substantial contact time between the source and the teeth of a user during cleaning.

It is possible that the calcium source of the present invention is provided in the form of a calcium phosphate. However, crystalline forms of calcium phosphate other than hydroxyapatite (HAP) may not be suitable precursors for the formation of HAP. Amorphous calcium phosphate (ACP) on the other hand has been found to be an excellent precursor for HAP formation when employed as small particles. Furthermore, so long as HAP is itself provided in the form of small particles, these have been found to be capable of assembling into enamel-like crystalline rods *in situ.* Thus if the calcium source of the present invention is a calcium phosphate then it is provided in the form of HAP and/or ACP having a weight average particle size of 5 microns or less (also referred to herein as "micronized HAP/ACP"). Preferably the particle size of the HAP and/or ACP is less than 2 microns, more preferably less than 1 micron, more preferably still less than 0.5 micron and most preferably in the range 0.01 to 0.1 micron.

Although the calcium source of the present invention, when present as calcium phosphate, is HAP and/or ACP with the particle size specified above, this does not preclude the presence of other calcium phosphate salts and/or larger particles of HAP or ACP. Preferably however, at least 50% by weight of calcium phosphate in the composition is made up by micronized HAP/ACP. More preferably the micronized HAP/ACP makes up at least 75% of the total amount of calcium phosphate in the composition, more preferably still at least 85% and most preferably the amount is in the range of 90 to 100%.

Additionally or alternatively the calcium source comprises a component capable of reacting with phosphate ions to produce a calcium phosphate *in situ.* Illustrative examples of the types of calcium source that may be used in this invention include, for example, calcium oxide, calcium silicate, calcium carbonate, calcium hydroxide, calcium sulfate, calcium carboxymethylcellulose, calcium alginate, bioactive glass, mixtures thereof or the like. In a preferred embodiment the calcium source is calcium silicate and/or bioactive glass. In a more preferred embodiment, the calcium silicate used is CaSiO₃ whereby the same is made commercially available under the name Microcal ET by PQ, Huber, Weifang Hongyuan Chemical Co., Ltd.

In yet another preferred embodiment, the calcium source is insoluble calcium silicate, present as the composite material calcium oxide-silica (CaO-SiO₂) as described in International patent application published as WO 2008/015117 (Unilever).

When a calcium silicate composite material is employed, the ratio of calcium to silicon (Ca:Si) may be from 1:10 to 3:1. The Ca:Si ratio is preferably from 1:5 to 2:1, and more preferably, from 1:3 to 2:1, and most preferably, from about 1:2 to 2:1. The calcium silicate may comprise mono-calcium silicate, bi-calcium silicate, or tri-calcium silicate whereby ratios of calcium to silicon (Ca:Si) should be understood to be atom ratios.

The calcium source employed in this invention may be in a crystalline or amorphous state, and preferably, the same is in an amorphous state. In an often preferred embodiment, the calcium source is in a mesoporous state, i.e. the source is a material having pores with diameters from 1 nm to 50 microns. Mesoporous calcium silicate (MCS) is often preferred.

The MCS which may be used in this invention can be made, for example, by combining a calcium salt, a silica precursor like silicate and a structure-directing agent to yield a solid suitable for calcinating. A more detailed description of the process that may be conducted to make a MCS suitable for use in this invention is described in the aforementioned International application, published as WO 2008/015117 and which is hereby incorporated by reference in its entirety.

Bioactive glass which may be used as the calcium source in the present invention comprises calcium and optionally phosphate ions. Suitable bioactive glasses are described, for example in WO 2010/041073 (BIOFILM LTD), WO 2009/158564 (NOVAMIN TECHNOLOGY INC), WO 99/13852 (UNIV MARYLAND), WO 2005/063185 (NOVAMIN TECHNOLOGY INC), WO 96/10985 (BIOXID OY) and/or WO 97/27148 (UNIV MARYLAND) all of which International patent applications are hereby incorporated by reference in their entirety.

Typically, the dentifrice composition of the present invention comprises from 0.1 to 60% by weight of the calcium source, more preferably from 0.2 to 50%. Even more preferably the dentifrice composition comprises the calcium source in an amount of at least 0.3% by weight, more preferably still at least 0.5% or even at least 1%. In a most preferred embodiment the dentifrice composition comprises the calcium source in an amount of at least 5% by weight, and optimally in the range 10 to 40% by weight.

Where the dentifrice is a tooth paste or powder, higher amounts of the calcium source are preferred. This is because tooth pastes are typically applied only in small volumes (e.g. around 2 ml) per consumer use. In addition tooth pastes are typically opaque and therefore allow for incorporation of high levels of insoluble or slightly soluble calcium sources without affecting the appearance expected by the consumer. Thus in one embodiment, the dentifrice is a tooth paste or powder and comprises the calcium source in an amount of at least 5% by weight, more preferably at least 7 % by weight and most preferably in the range 10 to 40% by weight.

Where the dentifrice is a mouth wash, lower amounts of the calcium source are preferred. This is because mouth washes are typically used in larger volumes (e.g. around 20 ml) per consumer use than tooth pastes. In addition mouth washes are typically transparent and therefore incorporation of high levels of insoluble or slightly soluble calcium sources may negatively affect the appearance expected by the consumer. Thus in one embodiment, the dentifrice is a mouth wash and comprises the calcium source in an amount of at least 0.2% by weight, more preferably at least 0.5% by weight and most preferably from 1 to 10% by weight.

Preferably the calcium source has a weight average particle size of five (5) microns or less, and preferably from 10 to 100%, and especially, from 25 to 100%, and most especially, from 70 to 100% by weight of the calcium source used in this invention has a particle size from 0.1 to 1.5 microns.

Further examples of calcium salts suitable for use in this invention are commercially available and often sold commercially from suppliers like Cole-Parmer, Great Lakes Calcium and Fujian Sannong Calcium Carbonate Co., Ltd.

The present inventors have surprisingly found that certain organic acids and their salts, namely organic acid having 1 to 3 carboxylic acid groups, enhance the generation of enamel-like HAP crystals on tooth surfaces. Without wishing to be bound by theory, the present inventors believe that the calcium source of this invention allows for the formation of nanoparticles of HAP on the tooth surface and that the organic acid promotes and regulates the aggregation and crystallization of the adsorbed nanoparticles into crystals which have the shape and orientation of native enamel. Further we believe that the carboxylate group of the organic acid is the key motif required for interaction with the HAP nanoparticles. Thus it is preferred that the organic acid has at least 2 carboxylic acid groups. Too many carboxylate groups may, however, cause the organic acid to chelate calcium ions and thus inhibit HAP formation and/or aggregation. Thus the organic acid has no more than 3 carboxylic acid groups.

The dentifrice composition comprises at least 0.1% by weight of organic acid having 1 to 3 carboxylic acid groups, or its physiologically acceptable salt, or a mixture thereof. Preferably the composition comprises the organic acid or its physiologically acceptable salt, or a mixture thereof in an amount in the range of 0.2 to 20% by weight, more preferably from 0.5 to 10% and most preferably from 1 to 5%.

The organic acid is an acidic amino acid. Suitable amino acids therefore include glutamic acid, aspartic acid or a mixture thereof and most preferred is glutamic acid.

Suitable physiologically acceptable salts of the organic acid include those in which the counterion is selected from the group consisting of Na⁺, K⁺, NH₄⁺, Ca²⁺, Mg²⁺, Al³⁺, Zn²⁺ and combinations thereof. Preferably the counterion is not calcium as this may affect the organic acid's ability to interact with HAP. Thus more preferably the counterion is selected from the group consisting of Na⁺, K⁺, NH₄⁺, Mg²⁺, Al³⁺, Zn²⁺ and combinations thereof. Even more preferable are salts with monovalent ions as these tend to be very water soluble. Thus most preferably the counterion is selected from the group consisting of Na⁺, K⁺, NH₄⁺ and combinations thereof.

The composition of the present invention is found to be capable of remineralisation of teeth *in situ* without inclusion of a phosphate source in the composition itself. Without wishing to be bound by theory the present inventors believe that this may be because the calcium source in the composition of the invention reacts with phosphate ions in saliva.

Thus in one embodiment the composition may be substantially free of phosphate source. This is especially preferred when the composition is a monophase hydrous composition (i.e. comprises greater than 1.5% water, preferably greater than 5% water, more preferably greater than 10% water and most preferably from 20 to 90% water by weight of the composition). Presence of both the calcium and phosphate sources in a monophase hydrous formulation can lead to premature reaction of the calcium and phosphate and instability of the product.

For certain compositions, especially anhydrous compositions (i.e. compositions substantially free from water) or dual phase hydrous compositions, it is preferable to compound a phosphate source in the composition to aid *in situ* generation of calcium phosphate.

The phosphate source that may be used in this invention is limited only to the extent that the same may be used in a composition suitable for use in an oral cavity. Illustrative examples of the types of phosphate source suitable for use in this invention include monosodium phosphate, sodium dihydrogenphosphate, disodium hydrogenphosphate, sodium pyrophosphate, tetrasodium pyrophosphate, sodium hexametaphosphate, monopotassium phosphate, potassium dihydrogenphosphate, dipotassium hydrogenphosphate, trisodium phosphate, tripotassium phosphate, mixtures thereof or the like. The phosphate source is preferably one which is water soluble.

Typically, the phosphate source makes up from 0.5 to 15%, and more preferably from 2 to 12%, and most preferably from 4 to 9% by weight of the dentifrice composition, based on total weight of the dentifrice composition and including all ranges subsumed therein. In a most preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

The dentifrice composition has a pH of greater than 6.0. If the pH of the composition is too low then it may lower the pH in the oral cavity such that generation of *in situ* calcium phosphate is retarded. In addition the stability of the calcium source in compositions with too low pH may be compromised. Therefore it is preferred that the pH is in the range 7.0 to 11.0, more preferably 8.0 to 10.5 and most preferably 8.5 to 10.0.

Surprisingly, the present inventors have also found that the efficacy of organic acid in facilitating growth orientation of newly formed apatite crystals can be even further improved by the presence of a fluoride source. Thus in a preferred embodiment the dentifrice composition comprises fluoride source. Suitable fluoride sources include sodium fluoride, stannous fluoride, sodium monofluorophosphate, zinc ammonium fluoride, tin ammonium fluoride, calcium fluoride, cobalt ammonium fluoride or mixtures thereof. The composition preferably comprises the fluoride source in an amount of at least 0.001%, and preferably, from 0.01 to 12%, and most preferably, from 0.1 to 5% by weight of the dentifrice composition, based on total weight of the dentifrice composition and including all ranges subsumed therein

The composition of the present invention is a dentifrice. Dentifrices comprise at least surfactant, thickener, humectant or a combination thereof.

Preferably the dentifrice composition comprises a surfactant. Preferably the composition comprises at least 0.01 % surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum Arabic, gum karaya, sodium alginate, carrageenan, guar, xanthan gum, Irish moss, starch, modified starch, silica based thickeners including silica aerogels, magnesium aluminum silicate (i.e., Veegum), Carbomers (cross-linked acrylates) and mixtures thereof.

Typically, sodium carboxymethyl cellulose and/or a Carbomer is/are preferred. When a Carbomer is employed, those having a molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

Thickener typically makes up from 0.01 to about 10%, and preferably, from 0.1 to 8%, and most preferably, from 1.5 to 6% by weight of the dentifrice composition, based on total weight of the composition and including all ranges subsumed therein.

When the dentifrice composition of this invention is a toothpaste or gel, the same typically has a viscosity from about 50,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the dentifrice composition. Preferably, the carrier humectant makes up from 25 to 80%, and most preferably, from 45 to 70% by weight of the dentifrice composition, based on total weight of the composition and including all ranges subsumed therein.

Dentifrice compositions described herein may comprise optional ingredients which are common in the art. These ingredients include antimicrobial agents, anti-inflammatory agents, anti-caries agents, plaque buffers, vitamins, plant extracts, desensitizing agents, anti-calculus agents, biomolecules, flavors, proteinaceous materials, preservatives, opacifying agents (especially titanium dioxide), coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the dentifrice composition described herein, and preferably, from 0.0 to 15% by weight, and most preferably, from 0.01 to 12% by weight of the composition, including all ranges subsumed therein.

The dentifrice composition of the present invention may be effective at whitening teeth even in the absence of oxidative whitening compound and in a preferred embodiment the composition is substantially free of oxidative whitening compound.

The dentifrice composition of this invention can be used in a method of remineralizing and/or whitening of teeth of an individual comprising the step of contacting one or more teeth of the individual with the dentifrice composition. The composition can additionally or alternatively be used in a method for the manufacture of a medicament for remineralizing and/or whitening of teeth of an individual comprising the step of contacting one or more teeth of the individual with the dentifrice composition.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 10 s to 10 hours, more preferably still from 30 s to 1 hour and most preferably from 1 minute to 5 minutes. The composition may be used daily, for example for use by an individual once, twice or three times per day.

### EXAMPLES

### Example 1

This example demonstrates the effect of glutamic acid on remineralisation of enamel with nanoparticles of hydroxyapatite (HAP).

### Experimental Section

All reagents were of analytical grade and used without further purification. Triply distilled CO₂-free water was used in the experiments. All solutions were filtered through 0.22 µm Millipore films prior to use.

Human third molars extracted without caries were used in this study. They were stored in a thymol solution after their roots and pulps were removed and were sectioned perpendicular to the dental crown using a slow speed diamond saw. The surfaces of each sample were polished using sillicon carbide paper in order remove the bacterium speckle and pigment, and etched in 37 wt% phosphoric acid for 10 s. Then these specimens were washed with de-ionized water and dried in air.

The apatite nanoparticles (-20-30 nm) were synthesized as described in Tao et al (J. Phys. Chem. B 2007, 111, 13410-13418). Then 0.1 wt% aqueous dispersion of the nanoparticle was prepared for further applications.

The simplified simulated body fluid (SBF) was prepared by dissolving NaCl, KCl, K₂HPO₄·3H₂O, MgCl₂·6H₂O, CaCl₂ into de-ionized water. The composition of SBF is shown in Table 1 (values are given in mM). The fluid was buffered at pH=7.40±0.05 at 37 °C with NaOH and HCl. No precipitation was observed during fluid preparation. When used, the concentration of glumatic acid (Glu) in SBF was 100 mM. The pH of these solutions was 7.40±0.05. All solutions were kept in water bath for 1h at 37 °C.

**TABLE 1**

| | ***Na***⁺ | ***K*⁺** | ***Ca*²⁺** | ***Mg*²⁺** | ***Cl*⁻** | ***HPO₄²⁻*** | ***HCO₃²⁻*** | ***SO₄²⁻*** |
|---|---|---|---|---|---|---|---|---|
| **SBF** | 142.0 | 5.0 | 2.5 | 1.5 | 147.8 | 1.0 | 4.2 | 0.5 |

Above-mentioned apatite nanoparticle dispersion with 3 *µ*L in volume was carefully dropped onto enamel surfaces and sample was dried in air at 25 °C. The enamel was further washed by 10 ml ethanol to remove weakly adsorbed particles and dried in air at 25 °C. Then these samples were immersed into SBF or SBF with glutamic acid for 72 h at 37 °C. Equivalent experiments were performed in the absence of nanoparticles of HAP.

Scanning electron microscopy (SEM) was performed by using a S-4800 field-emission scanning electron microscope (HITACHI, Japan) at an acceleration voltage of 5 kV.

The phase and orientation of newly formed layer was examined by X'PERT PRO X-ray diffractometer (PANalytical, Netherlands) with Cu *K*_{α} radiation with thin film mode.

FTIR spectra (Nicolet, Nexus670, USA) were used for crystallinity and adsorbed organic species identification.

Mechanical properties measurement was performed by means of a nanoindenter (Nanoindenter, XP, MTS, USA) with Berkovich tip (tip radius of about 20 nm).

### Results

Table 2 summarizes the results from the various tests.

**TABLE 2**

| **Substrate** | **Soaking Medium** | **New layer visible in SEM after soaking?** | **Structural orientation of new layer from SEM & XRD.** | **Hardness after soaking (GPa)*** |
|---|---|---|---|---|
| Enamel | SBF | Yes | Random | 2.2 ± 0.4 |
| Enamel + HAP nanoparticles | SBF | Yes | Random | Not Measured |
| Enamel | SBF + Glu | No | -------- | Not Measured |
| Enamel + HAP nanoparticles | SBF +Glu | Yes | Enamel-like | 4.7 ± 0.2 |

| | | | | |
|---|---|---|---|---|
| *Hardness of native enamel was 4.2 ± 0.2 GPa. | | | | |

As can be seen from the data in Table 2, soaking enamel for 72 hours in SBF resulted in formation of a new layer on the enamel surface but the structure of the deposited crystals was flake-like and not the rod-like crystals of native enamel. Furthermore the flake-like crystals had poor mechanical properties. Inclusion of glutamic acid in the SBF actually resulted in no deposition of a new crystalline layer on the enamel surface. In fact only when both nanoparticles of HAP and the glutamic acid were present could enamel-like crystals be formed on the enamel surface. In addition this new enamel-like layer was 0.5-1 micron thick and was at least as hard as native enamel.

### Example 2

This example demonstrates the effect of glutamic acid on remineralisation of enamel with particles of calcium silicate (CS).

### Experimental Section

Enamel blocks were incubated for 24 hours in SBF at 37 °C. CS was added to the SBF in an amount of 1 mg/ml. For some samples 100 mM of glutamic acid was also added to the SBF. Other conditions were similar to those described in Example 1.

### Results

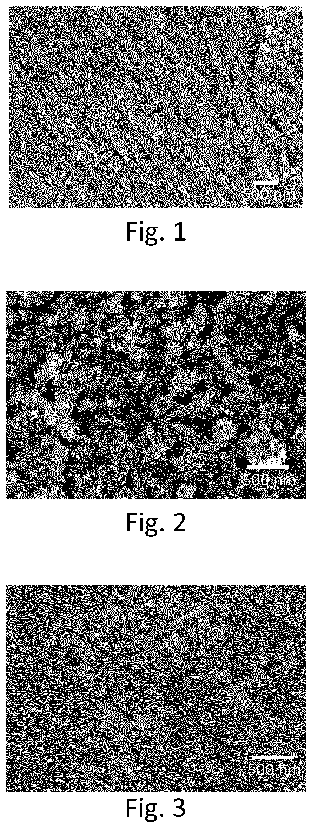

Figs 2 and 3 show SEM images of the morphology of the enamel surface after incubation with or without CS. The exposed enamel rods (Fig. 1) were covered by newly formed calcium phosphate particles. For samples incubated without CS (Fig. 2), the particles of calcium phosphate deposited on the enamel surface were small, spherical-like and randomly distributed on the surface. However, for enamel blocks incubated with CS and 100 mM Glu (Fig. 3), the morphology of the newly formed apatite was different. This layer was formed from rod-like small particles, tightly arranged on the surface and similar to native enamel surface morphology. Thus glutamic acid and calcium silicate can cooperatively remineralize enamel.

### Example 3

This example demonstrates the effect of glutamic acid and fluoride on remineralisation of enamel with particles of calcium silicate (CS).

### Experimental Section

Enamel blocks were brushed with a dual phase calcium silicate and phosphate toothpaste having the composition shown in Table 3.

**TABLE 3**

| **Dual-phase toothpaste** | | | |
|---|---|---|---|
| **CS Phase** | | **Phosphate Phase** | |
| **Component** | **Content (wt%)** | **Composition** | **Content (wt%)** |
| Chlorinated water | 35.89 | Deionized Water | 3.768 |
| Sweetener | 0.2 | PEG1500 | 2 |
| Potassium Nitrate | 0.5 | Sweetener | 0.27 |
| Sorbitol(70%) | 20.00 | NaH₂PO₄ | 6.41 |
| Benz. Alcohol | 0.5 | Na₃PO₄ | 7.64 |
| Calcium silicate | 30 | Sorbitol(70%) | 55 |
| Abrasive silica | 4 | Silica Abrasive | 12.00 |
| SCMC | 0.3 | Thickening Silica | 3.5 |
| Sodium Monofluorophosphate | 1.11 | Sodium Lauryl Sulphate | 6.6 |
| Flavor | 0.9 | Flavour | 1.20 |
| Sodium Lauryl Sulphate | 6.6 | SCMC | 0.5 |
| | | Sodium Monofluorophosphate | 1.11 |
| | | Colour | 0.002 |

3 g of toothpaste was prepared by mixing 1.5 g of each phase. 6 g of water was then added and slurried with the toothpaste as quickly as possible by hand-mixing (within 15 s). Immediately after preparation, the slurry was added within 5 s to the enamel blocks. Then the blocks were hand brushed for 1 min before incubating in the slurry for another 1 minute.

After brushing, the slurry was quickly removed from the blocks. The brushed blocks were washed by distilled water for 2 times using each time 15 ml of distilled water. Between brushes, the rinsed tooth samples were incubated in a shaking water bath at 37°C in 2 ml SBF. The SBF contained 100 mM glutamic acid, with or without 0.01 mM sodium fluoride.

### Results

After incubating with SBF containing glutamic acid for 4 days, enamel-like apatite rods formed on the surface of enamel. For the enamel blocks incubated with SBF and 100 mM glutamic acid but without fluoride, the newly formed apatite on the surface was in the form of randomly arranged rod crystals. For samples incubated with glutamic acid and 0.01 mM fluoride in SBF, the apatite rods formed on the surface were more oriented and the new layer was more ordered. Thus there was a cooperative effect of amino acid and fluoride on the growth orientation of apatite crystals (newly formed from CS) which was better than amino acid alone.

### Example 4

This example demonstrates the effect of toothpastes containing calcium silicate and various organic acids on tooth remineralisation.

### Experimental Section

Anhydrous and hydrous mono-phase toothpaste formulations were prepared as shown in Table 4.

**TABLE 4**

| **Component (%w/w)** | **Anhydrous Toothpaste** | **Hydrous Toothpaste** |
|---|---|---|
| Glycerine | To 100 | --- |
| PEG 400 | 10.50 | --- |
| Flavour | 1.20 | 1.00 |
| Trisodium Phosphate | 3.80 | --- |
| Calcium Silicate | 15.00 | 30.00 |
| Sodium Monofluorophosphate | 1.11 | 1.11 |
| Sweetener | 0.20 | 0.20 |
| Organic Acid | 0, 1.00 or 2.00 | 0, 1.00 or 2.00 |
| Monosodium Phosphate | 3.20 | --- |
| PEG 3000 | 1.75 | --- |
| Pigment | 0.05 | --- |
| Abrasive Silica | 7.00 | --- |
| Sodium Lauryl Sulphate | 2.00 | 2.20 |
| Sorbitol (70%) | --- | 20.0 |
| Water | --- | To 100 |
| Benz. Alcohol | --- | 0.50 |
| Potassium Nitrate | --- | 0.50 |
| Thickening Silica | --- | 1.00 |
| SCMC | --- | 0.30 |

In treatment, the teeth (bovine enamel blocks) were hand brushed with diluted toothpaste (toothpaste: water, 3 g: 6 g) for 1 min and incubated in toothpaste-water slurry for 1 min after brushing. During the following rinse off stage, each sample was rinsed with deionised water two times (15 ml each rinse). Brushing was performed 3 times per day for four weeks and between brushes samples were stored in SBF.

The organic acids tested were glutamic acid, glycine and citric acid. All toothpastes had a pH of about 9 regardless of presence of organic acid.

### Results

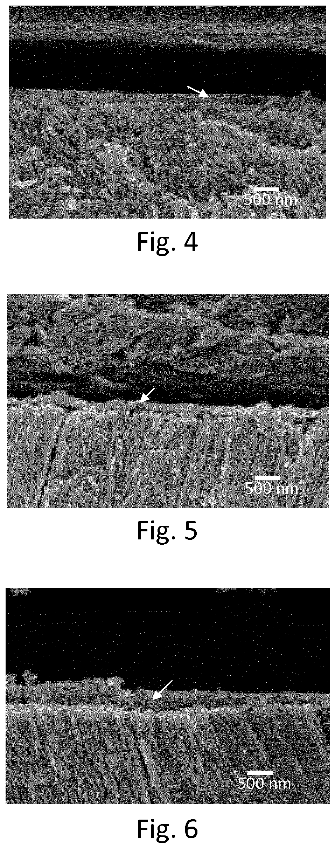

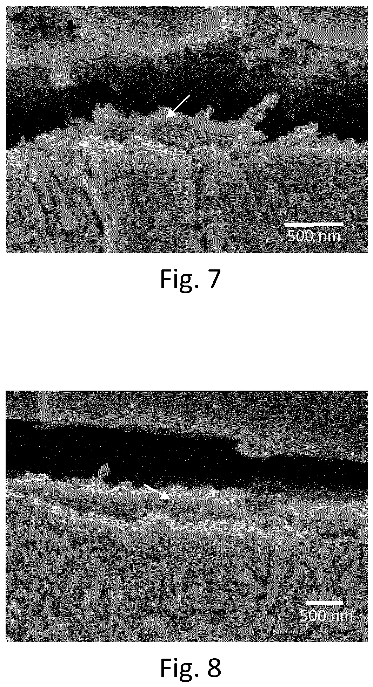

Figs. 4 to 8 show representative SEM images of the enamel blocks after two weeks brushing with anhydrous toothpaste. Location of any remineralized layer is indicated by a white arrow. A new layer was visible for blocks brushed with toothpaste without organic acid (Fig. 4). However, 1 % of glutamic acid (Fig. 5), glycine (Fig. 7) or citric acid (Fig. 8) improved layer thickness. The best coverage and thickness was apparent in samples brushed with toothpaste containing glutamic acid. Increasing the organic acid content of the toothpaste to 2% provided even better remineralisation (Fig. 6).

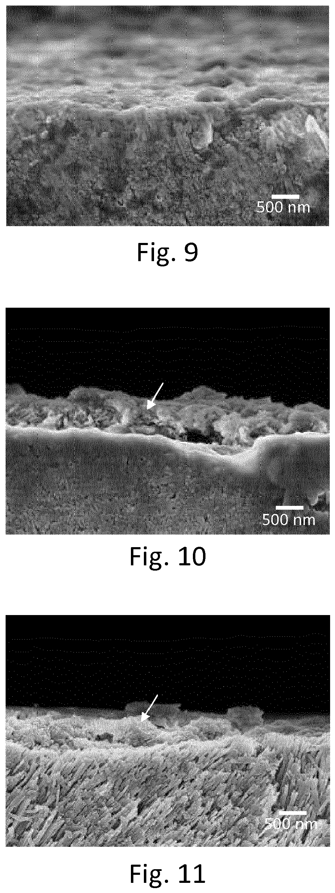

Figs. 9 to 11 show representative SEM images of the enamel blocks after four weeks brushing with hydrous toothpaste. Deposited particles were apparent on the surface of the enamel blocks brushed with toothpaste without organic acid (Fig. 9). Brushing with toothpaste containing the organic acids glycine (Fig. 10) or glutamic acid (Fig. 11) produced a thicker layer.

## Claims

1. A dentifrice composition comprising:
(a) water insoluble and/or slightly soluble calcium source, wherein the calcium source comprises
i. a component capable of reacting with phosphate ions to produce a calcium phosphate *in situ,*
ii. hydroxyapatite and/or amorphous calcium phosphate having a weight average particle size of 5 microns or less, or
iii. a combination thereof;
(b) at least 0.1 % by weight of organic acid or its physiologically acceptable salt, or a combination thereof, wherein the organic acid is acidic amino acid; and
(c) surfactant, thickener, humectant or a combination thereof;
wherein the dentifrice composition has a pH of greater than 6.0; and
wherein the component capable of reacting with phosphate ions to produce a calcium phosphate *in situ* comprises calcium oxide, calcium silicate, calcium carbonate, calcium hydroxide, calcium sulfate, calcium carboxymethylcellulose, calcium alginate, bioactive glass or a mixture thereof.

2. The dentifrice composition as claimed in claim 1, wherein the composition comprises a surfactant.

3. The dentifrice composition as claimed in claim 1 or claim 2, wherein the calcium source is water insoluble, preferably the calcium source is calcium silicate, bioactive glass or a combination thereof.

4. The dentifrice composition as claimed in claim 3, wherein the calcium source is calcium silicate having a ratio of Ca:Si in the range 1:10 to 3:1.

5. The dentifrice composition as claimed in any one of the preceding claims, wherein the composition comprises the calcium source in an amount of from 0.1 to 50% by weight.

6. The dentifrice composition as claimed in claim 5, wherein the dentifrice is a tooth paste or powder and comprises the calcium source in an amount of at least 5% by weight.

7. The dentifrice composition as claimed in claim 5, wherein the dentifrice is a mouth wash and comprises the calcium source in an amount of at least 0.5% by weight.

8. The dentifrice composition as claimed in any one of the preceding claims, wherein the organic acid is glutamic acid, aspartic acid or a mixture thereof.

9. The dentifrice composition as claimed in any one of the preceding claims, wherein the organic acid has 2 or 3 carboxylic acid groups, preferably the organic acid is glutamic acid.

10. The dentifrice composition as claimed in any one of the preceding claims, wherein the organic acid has a molar mass of less than 500 g mol⁻¹.

11. The dentifrice composition as claimed in any one of the preceding claims, wherein the composition comprises the organic acid in an amount in the range of 0.2 to 20% by weight.

12. The dentifrice composition as claimed in any one of the preceding claims, wherein the composition comprises a phosphate source, preferably the phosphate source is monosodium phosphate, sodium dihydrogenphosphate, disodium hydrogenphosphate, sodium pyrophosphate, tetrasodium pyrophosphate, sodium hexametaphosphate, monopotassium phosphate, potassium dihydrogenphosphate, dipotassium hydrogenphosphate, trisodium phosphate, tripotassium phosphate or a mixture thereof.

13. The dentifrice composition as claimed in any one of the preceding claims, wherein the pH is in the range of 7.0 to 11.0.

14. A dentifrice composition as claimed in any one of the preceding claims for use in remineralizing and/or whitening of teeth of an individual comprising the step of contacting one or more teeth of the individual with the dentifrice composition.

## Patentansprüche

1. Zahnpflegemittelzusammensetzung, umfassend:
(a) eine wasserunlösliche und/oder schwach lösliche Calciumquelle, wobei die Calciumquelle umfasst
i. eine Komponente, die im Stande ist, mit Phosphationen zu reagieren, um *in situ* ein Calciumphosphat zu bilden,
ii. Hydroxyapatit und/oder amorphes Calciumphosphat einer gewichtsgemittelten Partikelgröße von 5 Micron oder weniger oder
iii. eine Kombination davon,
(b) mindestens 0,1 Gewichts-% organische Säure oder ihres physiologisch verträglichen Salzes oder einer Kombination davon, wobei die organische Säure saure Aminosäure darstellt, und
(c) Tensid, Verdickungsmittel, Feuchthaltemittel oder eine Kombination davon,
wobei die Zahnpflegemittelzusammensetzung einen pH von größer als 6,0 aufweist und
wobei die Komponente, die im Stande ist, mit Phosphationen zu reagieren, um *in situ* Calciumphosphat zu bilden, Calciumoxid, Calciumsilikat, Calciumcarbonat, Calciumhydroxid, Calciumsulfat, Calciumcarboxymethylcellulose, Calciumalginat, bioaktives Glas oder eine Mischung davon umfasst.

2. Zahnpflegemittelzusammensetzung, wie im Anspruch 1 beansprucht, wobei die Zusammensetzung ein Tensid umfasst.

3. Zahnpflegemittelzusammensetzung, wie im Anspruch 1 oder Anspruch 2 beansprucht, wobei die Calciumquelle wasserunlöslich ist, vorzugsweise die Calciumquelle Calciumsilikat, bioaktives Glas oder eine Kombination davon darstellt.

4. Zahnpflegemittelzusammensetzung, wie im Anspruch 3 beansprucht, wobei die Calciumquelle Calciumsilikat mit einem Verhältnis von Ca:Si in dem Bereich von 1:10 bis 3:1 darstellt.

5. Zahnpflegemittelzusammensetzung, wie in irgendeinem der vorhergehenden Ansprüche beansprucht, wobei die Zusammensetzung die Calciumquelle in einer Menge von 0,1 bis 50 Gewichts-% umfasst.

6. Zahnpflegemittelzusammensetzung, wie im Anspruch 5 beansprucht, wobei das Zahnpflegemittel eine Zahnpasta oder ein Zahnpulver darstellt und die Calciumquelle in einer Menge von mindestens 5 Gewichts.-% umfasst.

7. Zahnpflegemittelzusammensetzung, wie im Anspruch 5 beansprucht, wobei das Zahnpflegemittel ein Mundspülmittel darstellt und die Calciumquelle in einer Menge von mindestens 0,5 Gewichts-% umfasst,

8. Zahnpflegemittelzusammensetzung, wie in irgendeinem der vorhergehenden Ansprüche beansprucht, wobei die organische Säure Glutaminsäure, Asparginsäure oder eine Mischung davon darstellt.

9. Zahnpflegemittelzusammensetzung, wie in irgendeinem der vorhergehenden Ansprüche beansprucht, wobei die organische Säure 2 oder 3 Carbonsäuregruppen aufweist, vorzugsweise die organische Säure Glutaminsäure ist.

10. Zahnpflegemittelzusammensetzung, wie in irgendeinem der vorhergehenden Ansprüche beansprucht, wobei die organische Säure eine Molmasse von weniger als 500 g mol⁻¹ aufweist.

11. Zahnpflegemittelzusammensetzung, wie in irgendeinem der vorhergehenden Ansprüche beansprucht, wobei die Zusammensetzung die organische Säure in einer Menge in dem Bereich von 0,2 bis 20 Gewichts-% umfasst.

12. Zahnpflegemittelzusammensetzung, wie in irgendeinem der vorhergehenden Ansprüche beansprucht, wobei die Zusammensetzung eine Phosphatquelle umfasst und die Phosphatquelle vorzugsweise Mononatriumphosphat, Natriumdihydrogenphosphat, Dinatriumhydrogenphosphat, Natriumpyrophosphat, Tetratnatriumpyrophosphat, Natriumhexametaphosphat, Monokaliumphosphat, Kaliumdihydrogenphosphat, Dikaliumhydrogenphosphat, Trinatriumphosphat, Trikaliumphosphat oder eine Mischung davon darstellt.

13. Zahnpflegemittelzusammensetzung, wie in irgendeinem der vorhergehenden Ansprüche beansprucht, wobei der pH in dem Bereich von 7,0 bis 11,0 liegt.

14. Zahnpflegemittelzusammensetzung, wie in irgendeinem der vorhergehenden Ansprüche beansprucht, zur Verwendung beim Remineralisieren und/oder Aufhellen von Zähnen eines Individuums, umfassend den Schritt des Inkontaktbringens eines oder mehrerer Zähne des Individuums mit der Zahnpflegemittelzusammensetzung.

## Revendications

1. Composition de dentifrice comprenant :
(a) une source de calcium insoluble et/ou légèrement soluble dans l'eau, où la source de calcium comprend
i. un constituant capable de réagir avec des ions phosphate pour produire un phosphate de calcium *in situ,*
ii. de l'hydroxyapatite et/ou du phosphate de calcium amorphe ayant une taille moyenne de particule en masse de 5 microns ou inférieure, ou
iii. une combinaison de ceux-ci ;
(b) au moins 0,1 % en masse d'acide organique ou de son sel physiologiquement acceptable, ou d'une combinaison de ceux-ci, où l'acide organique est un acide aminé acide ; et
(c) un tensioactif, épaississant, humectant ou une combinaison de ceux-ci ;
où la composition de dentifrice présente un pH supérieur à 6,0 ; et
où le constituant capable de réagir avec des ions phosphate pour produire un phosphate de calcium *in situ* comprend de l'oxyde de calcium, du silicate de calcium, du carbonate de calcium, de l'hydroxyde de calcium, du sulfate de calcium, de la calcium carboxyméthylcellulose, de l'alginate de calcium, du verre bioactif ou un mélange de ceux-ci.

2. Composition de dentifrice selon la revendication 1, où la composition comprend un tensioactif.

3. Composition de dentifrice selon la revendication 1 ou la revendication 2, où la source de calcium est insoluble dans l'eau, la source de calcium est de préférence du silicate de calcium, du verre bioactif ou une combinaison de ceux-ci.

4. Composition de dentifrice selon la revendication 3, où la source de calcium est du silicate de calcium ayant un rapport de Ca:Si dans l'intervalle de 1:10 à 3:1.

5. Composition de dentifrice selon l'une quelconque des revendications précédentes, où la composition comprend la source de calcium dans une quantité de 0,1 à 50 % en masse.

6. Composition de dentifrice selon la revendication 5, où le dentifrice est une pâte ou une poudre dentaire et comprend la source de calcium dans une quantité d'au moins 5 % en masse.

7. Composition de dentifrice selon la revendication 5, où le dentifrice est une eau de bouche et comprend la source de calcium dans une quantité d'au moins 0,5 % en masse.

8. Composition de dentifrice selon l'une quelconque des revendications précédentes, où l'acide organique est l'acide glutamique, l'acide aspartique ou un mélange de ceux-ci.

9. Composition de dentifrice selon l'une quelconque des revendications précédentes, où l'acide organique présente 2 ou 3 groupes acide carboxylique, l'acide organique est de préférence l'acide glutamique.

10. Composition de dentifrice selon l'une quelconque des revendications précédentes, où l'acide organique présente une masse molaire inférieure à 500 g mol⁻¹.

11. Composition de dentifrice selon l'une quelconque des revendications précédentes, où la composition comprend l'acide organique dans une quantité dans l'intervalle de 0,2 à 20 % en masse.

12. Composition de dentifrice selon l'une quelconque des revendications précédentes, où la composition comprend une source de phosphate, la source de phosphate est de préférence le phosphate de monosodium, dihydrogénophosphate de sodium, hydrogénophosphate de disodium, pyrophosphate de sodium, pyrophosphate de tétrasodium, hexamétaphosphate de sodium, phosphate de monopotassium, dihydrogénophosphate de potassium, hydrogénophosphate de dipotassium, phosphate de trisodium, phosphate de tripotassium ou un mélange de ceux-ci.

13. Composition de dentifrice selon l'une quelconque des revendications précédentes, où le pH se trouve dans l'intervalle de 7,0 à 11,0.

14. Composition de dentifrice selon l'une quelconque des revendications précédentes pour une utilisation dans une reminéralisation et/ou un blanchiment de dents d'un individu comprenant l'étape de mise en contact d'une ou plusieurs dents de l'individu avec la composition de dentifrice.
